# EUROPEAN PATENT SPECIFICATION

(11) **EP 3 038 616 B1**
(45) Date of publication and mention of the grant of the patent: **05.04.2023**
(21) Application number: 14841276.0
(22) Date of filing: 26.08.2014
(51) Int. Cl.: A61K 38/14, A61P 31/12, A61P 29/00

(54) **METHODS FOR TREATING BACTEREMIA AND OSTEOMYELITIS USING ORITAVANCIN**
VERFAHREN ZUR BEHANDLUNG VON BAKTERÄMIE UND OSTEOMYELITIS MIT ORITAVANCIN
MÉTHODES DE TRAITEMENT DE LA BACTÉRIÉMIE ET DE L'OSTÉOMYÉLITE À L'AIDE D'ORITAVANCINE

(30) Priority: 26.08.2013 US 201361869823 P
(43) Date of publication of application: 06.07.2016
(73) Proprietor: Melinta Therapeutics, Inc., Morristown, NJ 07960 (US)
(72) Inventor: MOECK, Gregory, St. Laurent, Québec H4L 2R3 (CA); ARHIN, Francis, Laval, Québec H7W 1S1 (CA)
(74) Representative: MacLean, Martin Robert
(86) International application number: PCT/US2014/052635
(87) International publication number: WO 2015/031313

(56) References cited:
- WO-A2-00/66144
- WO-A2-2010/025438
- P. G. AMBROSE ET AL: "In Vivo Activity of Oritavancin in Animal Infection Models and Rationale for a New Dosing Regimen in Humans", CLINICAL INFECTIOUS DISEASES, vol. 54, no. suppl 3, 19 March 2012 (2012-03-19), pages S220-S228, XP055358792, US ISSN: 1058-4838, DOI: 10.1093/cid/cis001
- G W Kaatz ET AL: "Efficacy of LY333328 against experimental methicillin-resistant Staphylococcus aureus endocarditis", Antimicrobial agents and chemotherapy, 1 April 1998 (1998-04-01), pages 981-983, XP055358793, UNITED STATES Retrieved from the Internet: URL:http://aac.asm.org/content/42/4/981.fu ll.pdf#page=1&view=FitH
- POULAKOU G ET AL: "Oritavancin: A new promising agent in the treatment of infections due to Gram-positive pathogens", EXPERT OPINION ON INVESTIGATIONAL DRUGS, INFORMA HEALTHCARE, UK, vol. 17, no. 2, 1 February 2008 (2008-02-01), pages 225-243, XP002707676, ISSN: 1354-3784, DOI: 10.1517/13543784.17.2.225
- TANAKA K S E ET AL: "Synthesis and in vitro evaluation of bisphosphonated glycopeptide prodrugs for the treatment of osteomyelitis", BIOORGANIC & MEDICINAL CHEMISTRY LETTERS, PERGAMON, AMSTERDAM, NL, vol. 20, no. 4, 15 February 2010 (2010-02-15), pages 1355-1359, XP026887069, ISSN: 0960-894X, DOI: 10.1016/J.BMCL.2010.01.006 [retrieved on 2010-02-03]
- G. P. Allen ET AL: "In Vitro Activities of Quinupristin-Dalfopristin and Cefepime, Alone and in Combination with Various Antimicrobials, against Multidrug-Resistant Staphylococci and Enterococci in an In Vitro Pharmacodynamic Model", ANTIMICROBIAL AGENTS AND CHEMOTHERAPY, vol. 46, no. 8, 1 August 2002 (2002-08-01) , pages 2606-2612, XP055562104, US ISSN: 0066-4804, DOI: 10.1128/AAC.46.8.2606-2612.2002

## Description

### TECHNICAL FIELD

The invention relates to a therapeutically-effective amount of oritavancin or a salt thereof for use in methods of treating bacterial infections in a subject as claimed, including bacteremia, osteomyelitis, and endocarditis.

### BACKGROUND

Oritavancin diphosphate (oritavancin; also known as N^{DISACC}-(4-(4-chlorophenyl)benzyl)A82846B and LY333328) is a semi-synthetic lipoglycopeptide derivative of a naturally occurring glycopeptide. Its structure confers potent antibacterial activity against gram-positive bacteria, including vancomycin-resistant enterococci (VRE), methicillin- and vancomycin-resistant staphylococci, and penicillin-resistant streptococci. The rapidity of its bactericidal activity against exponentially-growing *S. aureus* (≥ 3-log reduction within 15 minutes to 2 hours against MSSA, MRSA, and VRSA) is one of the features that distinguishes it from the prototypic glycopeptide vancomycin (McKay et al., J Antimicrob Chemother. 63(6):1191-9 (2009), Epub 2009 Apr 15).

Oritavancin inhibits the synthesis of peptidoglycan, the major structural component of the bacterial cell wall by a mechanism that is shared with glycopeptides, such as vancomycin (Allen et al., Antimicrob Agents Chemother 41(1):66-71 (1997); Cegelski et al., J Mol Biol 357:1253-1262 (2006); Arhin et al., Poster C1-1471: Mechanisms of action of oritavancin in Staphylococcus aureus [poster]. 47th Intersci ConfAntimicro Agents Chemo, Sept. 17-20, 2007; Chicago, IL). Oritavancin, like vancomycin, binds to the Acyl-D-Alanyl-D-Alanine terminus of the peptidoglycan precursor, lipid-bound N-acetyl-glucosamine-N-acetyl-muramic acid-pentapeptide (Reynolds, Eur J Clin Microbiol Infect Dis 8(11):943-950 (1989); Nicas and Allen, Resistance and mechanism of action. In: Nagarajan R, editor. Glycopeptide antibiotics. New York: Marcel Dekker 195-215 (1994); Allen et al., Antimicrob Agents Chemother 40(10):2356-2362 (1996); Allen and Nicas, FEMS Microbiology Reviews 26:511-532 (2003); Kim et al., Biochemistry 45:5235-5250 (2006)). However, oritavancin inhibits cell wall biosynthesis even when the substrate is the altered peptidoglycan precursor that is present in VRE and vancomycin-resistant *S. aureus* (VRSA). Thus, the spectrum of oritavancin antibacterial activity extends beyond that of vancomycin to include glycopeptide-resistant enterococci and staphylococci (Ward et al., Expert Opin Investig Drugs 15:417-429 (2006); Scheinfeld, J Drugs Dermatol 6:97-103 (2007)). Oritavancin may inhibit resistant bacteria by interacting directly with bacterial proteins in the transglycosylation step of cell wall biosynthesis (Goldman and Gange, Curr Med Chem 7(8):801-820 (2000); Halliday et al., Biochem Pharmacol 71(7):957-967 (2006); Wang et al., Poster C1-1474: Probing the mechanism of inhibition of bacterial peptidoglycan glycotransferases by glycopeptide analogs. 47th Intersci Conf Antimicro Agents Chemo, Sept. 17-20, 2007). Oritavancin also collapses transmembrane potential in gram positive bacteria, leading to rapid killing (McKay et al., Poster C1-682: Oritavancin disrupts transmembrane potential and membrane integrity concomitantly with cell killing in Staphylococcus aureus and vancomycin-resistant Enterococci. 46th Intersci Conf Antimicro Agents Chemo, San Francisco, CA, Sept. 27-30, 2006). These multiple effects contribute to the rapid bactericidal activity of oritavancin. Tanaka et al. (Bioorganic and medicinal chemistry letters, 2010, 20(4), 1355-1359) describes bisphosphonated prodrugs of oritavancin and their possible use to treat osteomyelitis. Ambrose et. al. (Clin. Infect. Dis. 2012 Apr;54 Suppl 3:S220-8) describes *in vivo* activity of oritavancin in animal infection models and rationale for a new dosing regimen in humans. Kaatz et. al. (Antimicrob Agents Chemother. 1998 Apr; 42(4): 981-983) describes efficacy of LY333328 against experimental methicillin-resistant staphylococcus aureus endocarditis. WO2010/025438A2 describes methods of treatment using single doses of oritavancin.

### BRIEF SUMMARY

The present invention is defined by the claims. The present disclosure describes a therapeutically-effective amount of oritava.ncin or a salt thereof for use in methods of treating bacteremia in a subject, said method comprising administering a therapeutically-effective amount of oritavancin or a salt thereof to a subject having bacteremia, thereby treating bacteremia in a subject. In certain aspects of this disclosure , (i) the bacteremia was resistant to previous antibiotic treatment, (ii) the subject was intolerant to other antibiotics, and/or (iii) the subject also has osteomyelitis or endocarditis, or both. In certain aspects of this disclosure , the bacteremia is caused by a Gram-positive organism, for example, a multi-drug resistant (MDR) strain of methicillin-resistant *Staphylococcus aureus* (MRSA). In certain aspects of this disclosure , the therapeutically-effective amount of oritavancin or a salt thereof is at least about 1200 mg. In certain aspects of this disclosure , treatment is achieved by administering a single, 1200 mg dose of oritavancin or a salt thereof to the subject.

In an embodiment, the invention is drawn to a therapeutically-effective amount of oritavan cin or a salt thereof for use in methods of treating bacteremia and osteomyelitis in a subject, said method comprising administering a therapeutically-effective amount of oritavancin or a salt thereof to a subject having bacteremia and osteomyelitis, thereby treating bacteremia and osteomyelitis in a subject. In certain aspects of this disclosure, (i) the bacteremia was resistant to previous antibiotic treatment, (ii) the subject was intolerant to other antibiotics, and/or (iii) the subject also has endocarditis. In certain aspects of this disclosure, the bacteremia is caused by a Gram-positive organism, for example, a multi-drug resistant (MDR) strain of methicillin-resistant *Staphylococcus aureus* (MRSA). In certain aspects of this disclosure, the therapeutically-effective amount of oritavancin or a salt thereof is at least about 1200 mg. In certain aspects of this disclosure, treatment is achieved by administering a single, 1200 mg dose of oritavancin or a salt thereof to the subject.

The present disclosure describes a thernpeutiailly-effective amount of oritavancin or a salt thereof for use in methods of treating bacteremia and endocarditis in a subject, said method comprising administering a therapeutically-effective amount of oritavancin or a salt thereof to a subject having bacteremia and endocarditis, thereby treating bacteremia and endocarditis in a subject. In certain aspects of this disclosure, (i) the bacteremia was resistant to previous antibiotic treatment, (ii) the subject was intolerant to other antibiotics, and/or (iii) the subject also has osteomyelitis. In certain aspects of this disclosure, the bacteremia is caused by a Gram-positive organism, for example, a multi-drug resistant (MDR) strain of methicillin-resistant *Staphylococcus aureus* (MRSA). In certain aspects of this disclosure, the therapeutically-effective amount of oritavancin or a salt thereof is at least about 1200 mg. In certain aspects of this disclosure, treatment is achieved by administering a single, 1200 mg dose of oritavancin or a salt thereof to the subject.

The present invention is defined by the claims.

### DETAILED DESCRIPTION

### I. Definitions

As used herein, "a" or "an" may mean one or more. As used herein when used in conjunction with the word "comprising," the words "a" or "an" may mean one or more than one. As used herein "another" may mean at least a second or more. Furthermore, unless otherwise required by context, singular terms include pluralities and plural terms include the singular.

As used herein, "about" refers to a numeric value, including, for example, whole numbers, fractions, and percentages, whether or not explicitly indicated. The term "about" generally refers to a range of numerical values (e.g., +/- 5-10% of the recited value) that one of ordinary skill in the art would consider equivalent to the recited value (e.g., having the same function or result). In some instances, the term "about" may include numerical values that are rounded to the nearest significant figure.

### II. The Present Invention

Oritavancin (also termed N-(4-(4-chlorophenyl)benzyl)A82846B and LY333328) has the following chemical structure:

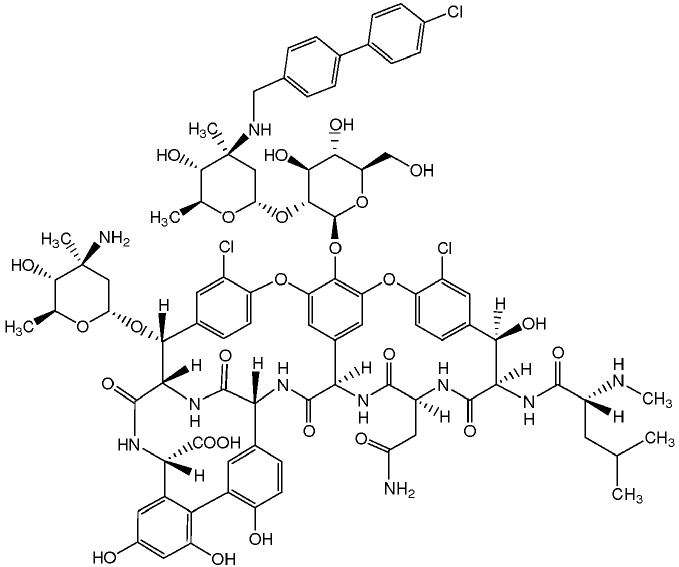

Oritavancin may be used *per se* in the methods **described herein**, or in the form of a pharmaceutically acceptable salt, hydrate, solvate, or mixtures thereof, and includes oritavancin diphosphate. The term "pharmaceutically acceptable salt" refers to non-toxic acid addition salts derived from inorganic and organic acids. While reference is made herein to both "oritavancin" and "a pharmaceutically acceptable salt thereof", the term "oritavancin" should be understood to include both the compound *per se* as well as a pharmaceutically acceptable salt, hydrate, solvate, or a mixture thereof, including oritavancin diphosphate, unless otherwise indicated by context, as the term "oritavancin" alone may be used for the sake of brevity.

Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenylsulfonic acid, carbonic acid, succinic acid, citric acid, benzoic acid, acetic acid, and the like. Base addition salts include those derived from inorganic bases, such as ammonium or alkali or alkaline earth metal hydroxides, carbonates, bicarbonates, and the like. Such bases useful in preparing the salts of this disclosure thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

It should be recognized that the particular counter-ion forming a part of any salt of this disclosure is not of a critical nature, so long as the salt as a whole is pharmacologically acceptable and as long as the counter-ion does not contribute undesired qualities to the salt as a whole.

Means for the preparation of the glycopeptide antibiotics, including oritavancin and analogs thereof, may be found, for example, in U.S. Patent No. 5,840,684

### Pharmaceutical Compositions

In each of the aspects, embodiments and methods of the present disclosure, oritavancin may be administrated to the subject in the form of a pharmaceutical composition. The pharmaceutical compositions of the disclosure comprise oritavancin, or a pharmaceutically acceptable salt, hydrate, solvate, or a mixture thereof, and a pharmaceutically acceptable carrier or excipient. In specific aspects, the pharmaceutical compositions of the disclosure comprise oritavancin, or a pharmaceutically acceptable salt thereof.

Pharmaceutically acceptable carriers and excipient are those compounds, solutions, substances or materials that can be used to produce formulations of oritavancin that are suitable for administered to a subject, such as a human. In particular, carriers and excipients of the present disclosure are those useful in preparing a pharmaceutical composition that is generally safe, non-toxic and neither biologically nor otherwise undesirable and that may present pharmacologically favorable profiles, and includes carriers and excipient that are acceptable for veterinary use as well as human pharmaceutical use. Suitable pharmaceutically acceptable carriers and excipients are well known in art and can be determined by those of skill in the art as the clinical situation warrants. The skilled artisan will understand that diluents are included within the scope of the terms carriers and excipients. Examples of suitable carriers and excipients include dextrose, water, glycerol, ethanol, propylene glycol, polysorbate 80 (Tween-80^{™}), poly(ethylene)glycol 300 and 400 (PEG 300 and 400), PEGylated castor oil (e.g. Cremophor EL), poloxamer 407 and 188, a cyclodextrin or a cyclodextrin derivative (including HPCD ((2-hydroxypropyl)-cyclodextrin) and (2-hydroxyethyl)-cyclodextrin; see, e.g., U.S. patent application publication 20060194717), hydrophilic and hydrophobic carriers, and combinations thereof. Hydrophobic carriers include, for example, fat emulsions, lipids, PEGylated phospholipids, polymer matrices, biocompatible polymers, lipospheres, vesicles, particles, and liposomes. The terms specifically exclude cell culture medium. More particularly: (1) 5% (w/v) dextrose, or (2) water (e.g., sterile water; Water-For-Infection), may be used as a pharmaceutically acceptable carrier.

Excipients included in a formulation have different purposes depending, for example on the nature of the drug, and the mode of administration. Examples of generally used excipients include, without limitation: stabilizing agents, solubilizing agents and surfactants, buffers, antioxidants and preservatives, tonicity agents, bulking agents, lubricating agents, emulsifiers, suspending or viscosity agents, inert diluents, fillers, disintegrating agents, binding agents, wetting agents, lubricating agents, antibacterials, chelating agents, sweeteners, perfuming agents, flavoring agents, coloring agents, administration aids, and combinations thereof.

The compositions may contain common carriers and excipients, such as cornstarch or gelatin, lactose, sucrose, microcrystalline cellulose, kaolin, mannitol, dicalcium phosphate, sodium chloride, alginic acid, croscarmellose sodium, and sodium starch glycolate.

The particular carrier, diluent or excipient used will depend upon the means and purpose for which the active ingredient is being applied.

Pharmaceutically acceptable excipients also include tonicity agents that make the composition compatible with blood. Tonicity agents are particularly desirable in injectable formulations.

Acceptable methods for preparing the pharmaceutical compositions according to the disclosure are known to those skilled in the art. For example, pharmaceutical compositions may be prepared following conventional techniques of the pharmaceutical chemist involving steps such as mixing, granulating, and compressing when necessary for tablet forms, or mixing, filling, and dissolving the ingredients as appropriate, to give the desired products for various routes of administration.

### Modes of Administration

The pharmaceutical compositions and compounds of the present disclosure may be formulated, for example, for oral, enteral, sublingual, intranasal, intraocular, rectal, intravaginal, transdermal, mucosal, topical or parenteral administration. Parenteral modes of administration include without limitation, intradermal, subcutaneous (s.c., s.q., sub-Q, Hypo), intramuscular (i.m.), intravenous (i.v.), intraperitoneal (i.p.), intra-arterial, intramedullary, intracardiac, intraarticular (joint), intrasynovial (joint fluid area), intracranial, intraspinal, and intrathecal (spinal fluids). Any known device useful for parenteral injection or infusion of drug formulations can be used to effect such administration. In certain aspects of each of the embodiments of the disclosure, the pharmaceutical composition is administered to the subject intravenously.

Formulations for parenteral administration can be in the form of aqueous or nonaqueous isotonic sterile solutions, suspensions or fat emulsions. The unit dosage of these solutions or suspensions can be in a concentrated liquid, powder or granular form for *ex tempore* reconstitution in the appropriate pharmaceutically acceptable carrier, such as sterile water, at the time of delivery. In addition to the above-mentioned excipients, powder forms optionally include bulking agents (e.g. mannitol, glycine, lactose, sucrose, trehalose, dextran, hydroxyethyl starch, ficoll and gelatin), and cryo or lyoprotectants. In an alternative embodiment, the parenteral unit dosage form of pharmaceutical compositions and compounds of the present disclosure can be a ready-to-use solution of the pharmaceutical compositions and compounds in a suitable carrier in sterile, hermetically sealed ampoules or in sterile pre-loaded syringes. The suitable carrier optionally comprises any of the above-mentioned excipients. The parenteral form used for injection must be fluid to the extent that easy syringability exists.

Excipients used in parenteral preparations may also include, without limitation, stabilizing agents (e.g. carbohydrates, amino acids and polysorbates, such as 5% dextrose), solubilizing agents (e.g. cetrimide, sodium docusate, glyceryl monooleate, polyvinylpyrolidone (PVP) and polyethylene glycol (PEG)), surfactants (e.g. polysorbates, tocopherol PEG succinate, poloxamer and Cremophor^{™}), buffers (e.g. acetates, citrates, phosphates, tartrates, lactates, succinates, amino acids and the like), antioxidants and preservatives (e.g. BHA, BHT, gentisic acids, vitamin E, ascorbic acid, sodium ascorbate and sulfur containing agents such as sulfites, bisulfites, metabisulfites, thioglycerols, thioglycolates and the like), tonicity agents (for adjusting physiological compatibility), suspending or viscosity agents, antibacterials (e.g. thimersol, benzethonium chloride, benzalkonium chloride, phenol, cresol and chlorobutanol), chelating agents, and administration aids (e.g. local anesthetics, anti-inflammatory agents, anti-clotting agents, vaso-constrictors for prolongation and agents that increase tissue permeability), and combinations thereof.

Parenteral formulations using hydrophobic carriers include, for example, fat emulsions and formulations containing lipids, lipospheres, vesicles, particles and liposomes. Fat emulsions include in addition to the above-mentioned excipients, a lipid and an aqueous phase, and additives such as emulsifiers (e.g. phospholipids, poloxamers, polysorbates, and polyoxyethylene castor oil), and osmotic agents (e.g. sodium chloride, glycerol, sorbitol, xylitol and glucose). Liposomes include natural or derived phospholipids and optionally stabilizing agents such as cholesterol.

In intravenous (IV) use, a sterile formulation of the pharmaceutical compositions of the present disclosure and optionally one or more additives, including solubilizers or surfactants, can be dissolved or suspended in any of the commonly used intravenous fluids and administered by infusion. Intravenous fluids include 5% dextrose in water.

In intramuscular preparations, a sterile formulation of the pharmaceutical compositions of the present disclosure can be dissolved and administered in a pharmaceutical diluent such as Water-for-Injection (WFI) or 5% dextrose in water. A suitable insoluble form of the pharmaceutical compositions may be prepared and administered as a suspension in an aqueous base or a pharmaceutically acceptable oil base, e.g. an ester of a long chain fatty acid such as ethyl oleate.

While oritavancin and pharmaceutical compositions can be administered in a systemic manner through the means described above, they may also be administered in a localized manner. For example, the active agents may be administered directly, such as through a topical composition or directly into a subcutaneous or other form of wound.

### Oritavancin or a Salt Thereof for Use in Methods of Treatment, Prophylaxis and Prevention

Oritavancin or a salt thereof for use in methods of the disclosure comprise contacting bacteria with an effective amount of oritavancin. For example, one can inhibit cell wall biosynthesis in a gram-positive bacterium by contacting such a bacterium with oritavancin. The contacting may be carried out *in vitro* (e.g., in biochemical and/or cellular assays), *in vivo* in a non-human animal, *in vivo* in mammals, including humans and/or *ex vivo* (e.g. for sterilization purposes). As used herein, the term "contacting" is meant to broadly refer to bringing a bacterial cell and a molecule of oritavancin into sufficient proximity such that oritavancin can exert an effect on the bacterial cell. Oritavancin may be transported to the location of the bacterial cell, or oritavancin may be situated in a location to which the bacterial cell travels or is brought into contact. The skilled artisan will understand that the term "contacting" includes physical interaction between oritavancin and a bacterial cell, as well as interactions that do not require physical interaction.

As used herein, a "subject" means an animal, such as a mammal, including humans, other higher primates, lower primates, and animals of veterinary importance, such as dogs, cats, horses, sheep, goats, and cattle and the like. The subject may have a bacterial infection, may be at risk for developing a bacterial infection, or may be at greater risk than the general population for exposure to infectious bacteria.

As used herein, "bacterial infection" refers to an infection caused by a species or strain of bacteria for which the methods disclosed herein are appropriate. For example, the methods of treatment may be used in the treatment of subjects having one or more of bacterial skin infections, such as complicated skin and skin structure infections (cSSSI), acute bacterial skin and skin structure infections (ABSSSI), and complicated and uncomplicated skin and soft tissue infections (SSTI), including abscesses, ulcers, burns and cellulitis. The oritavancin or a salt thereof for use in methods of treatment also include treatment of one or more of deep bacterial infections, such as major abscess, infected ulcer, major burn, or deep and extensive cellulitis. Further bacterial infections that may be treated using a therapeutically-effective amount of oritavancin or a salt thereof for use in the methods of the present disclosure include one or more of bacteremia, i.e., blood stream infections (BSI), catheter-related blood stream infections (CRBSI), endocarditis, osteomyelitis, prosthetic joint infections, pneumonia (community acquired and nosocomial), joint space infections and device infections (e.g., infections associated with pace makers and internal cardiac defibrillators). The oritavancin or a salt thereof for use in methods of treatment can also be practiced concomitantly with surgical intervention for the bacterial infection.

The infectious bacteria and those bacteria causing bacterial infections (including, but not limited to, bacteremia, osteomyelitis and endocarditis) that may be treated or prevented via the compositions and methods of the present disclosure include those described in U.S. Patent No. 5,840,684, gram-positive bacteria, and in particular, *Staphylococcus aureus* (methicillin-susceptible and -resistant strains; vancomycin-susceptible, -intermediate, -hetero-intermediate and -resistant strains), *Streptococcus pyogenes, Streptococcus agalactiae, Streptococcus anginosus* grp. (including *S*. *anginosus, S. intermedius,* and *S. constellatus), Streptococcus dysgalactiae* (including *S. dysgalactiae* subsp. *equisimilis), Streptococcus pneumoniae,* Streptococci species, including Streptococci Group A species, Streptococci Group B species, Streptococci Group C species, and Streptococci Group D species, Enterococci species, *Enterococcus faecalis* (vancomycin-susceptible and -resistant strains), *Enterococcus faecium* (vancomycin-susceptible and -resistant strains), *Staphylococcus epidermidis* (methicillin-susceptible and -resistant strains), *Staphylococcus haemolyticus, Bacillus anthracis,* and *Clostridium difficile* (both vegetative form and spores).

Administration frequencies for the pharmaceutical compositions of the present disclosure will vary based on the method being practiced, the physical characteristics of the subject, the severity of the subject's symptoms, the form of the infection, the identity of the bacteria, and the formulation and the means used to administer the drug. However, administration frequencies will generally include 4, 3, 2 or once daily, every other day, every third day, every fourth day, every fifth day, every sixth day, once weekly, every eight days, every nine days, every ten days, bi-weekly, monthly and bi-monthly. In certain aspects, the pharmaceutical composition is administered once daily. The duration of treatment will be based on the condition being treated and will be best determined by the attending physician. Under some conditions, treatment will be continued for a number of days, weeks, or months. Under other conditions, complete treatment will be achieve through administering one, two or three dose of the pharmaceutical composition over the entire course of treatment. In certain aspects, complete treatment can be achieved using a single dose of the pharmaceutical composition.

A second therapeutic agent may be administered to the subject. Such second therapeutic agents may be included in a pharmaceutical formulation comprising oritavancin, or they may be administered separately, whether concurrently or sequentially, in either order. A wide range of second therapeutic agents, such as antibiotics, can be used in combination with the compounds, compositions and methods of the present disclosure. Antibiotics used as second therapeutic agents may act by interfering with cell wall synthesis, plasma membrane integrity, nucleic acid synthesis, ribosomal function, folate synthesis, etc. A non-limiting list of useful antibiotics includes: fusidic acid, trimethoprim, sulfadiazine, sulfamethoxazole, a penicillin, a monobactam, a penam, a penem, a clavam, a clavem, a carbopenam, a carbopenem, a cepham, a cephem, an oxacepham, an oxacephem, a carbocepham, a carbocephem, a cephalosporin, tetracycline, a tetracycline derived antibacterial agent, glycylcycline, a glycylcycline derived antibacterial agent, minocycline, a minocycline derived antibacterial agent, sancycline, a sancycline derived antibacterial agent, methacycline, a methacycline derived antibacterial agent, an oxazolidinone antibacterial agent, an aminoglycoside antibacterial agent, an additional glycopeptide or lipoglycopeptide, a quinolone antibacterial agent, daptomycin, a daptomycin derived antibacterial agent, rifamycin, a rifamycin derived antibacterial agent, rifampin, a rifampin derived antibacterial agent, rifalazil, a rifalazil derived antibacterial agent, rifabutin, a rifabutin derived antibacterial agent, rifapentin, a rifapentin derived antibacterial agent, rifaximin and a rifaximin derived antibacterial agent. The second therapeutic agent may be administered before, concurrently with, or after a pharmaceutical formulation of the present disclosure is administered to a subject.

### Oritavancin or a Salt Thereof for Use in Methods of Treating

As discussed in the summary of the disclosure above, atherapeutically-effectiveamountoforitavancin or a salt thereof for use in methods of treating one or more of bacteremia, osteomyelitis, or endocarditis in a subject is described herein. Thus, the disclosure includes a therapeutically-effective amount of oritavancin or a salt thereof for use in methods of treating bacteremia, osteomyelitis, or endocarditis, comprising administering to a subject having bacteremia, osteomyelitis, or endocarditis a therapeutically effective amount of a pharmaceutical composition comprising oritavancin, or a pharmaceutically acceptable salt thereof, and a pharmaceutically acceptable carrier or diluent, thereby treating bacteremia, osteomyelitis, or endocarditis in a subject. In an aspect of this disclosure, a second therapeutic agent may be administered to the subject in conjunction with the pharmaceutical composition comprising oritavancin. In certain aspects, the disclosure is drawn to a therapeutically-effective amount of oritavancin or a salt thereof for use in methods of treating a subject having bacteremia. In other aspects, the disclosure is drawn to a therapeutically-effective amount of oritavancin or a salt thereof for use in methods of treating a subject having both bacteremia and osteomyelitis, methods of treating a subject having both bacteremia and endocarditis, and methods of treating a subject having each of bacteremia, osteomyelitis and endocarditis. The subjects of this disclosure may be further defined as having one or more of the following characteristics: the bacteremia was resistant to previous antibiotic treatments and the subject was intolerant to other antibiotics.

The terms "treating" and "treatment" mean at least the mitigation of a bacterial infection, or a disease condition or symptom associated with a bacterial infection, in a subject that is achieved by a reduction of growth, replication, and/or propagation, or death or destruction of bacteria, on or in the subject. The terms "treating" and "treatment" include curing, healing, inhibiting, relieving from, improving and/or alleviating, in whole or in part, the bacterial infection or associated disease condition or symptom. The mitigation of a bacterial infection or associated disease condition or symptom may be about 100%, 99%, 98%, 97%, 96%, 95%, 90%, 80%, 70%, 60%, 50%, 40%, 30%, 20%, 10%, 5% or 1% in the subject, versus a subject to which oritavancin has not been administered. In one aspect, treating means reducing the population of bacteria causing the infection in the subject to an undetectable level, where detection is by any conventional means, such culturing a sample in the laboratory. In another aspect, treating means complete healing of the infection, shown by an absence of clinical symptoms associated with the infection. In a further aspect, treating means the mitigation of a bacterial infection or associated disease condition or symptom by at least about 90% in the subject. In an additional aspect, treating means the mitigation of a bacterial infection or associated disease condition or symptom by at least about 95% in the subject.

The therapeutically effective amount of oritavancin and the amount sufficient to achieve the stated goals of a therapeutically-effective amount of oritavancin or a salt thereof for use in the methods of treatment disclosed herein in each dosage will vary, for example, in view of the physical characteristics of the subject, the severity of the subject's, symptoms, the form of the infection, the identity of the bacteria, the formulation and the means used to administer the drug, and the method being practiced. The specific dose for a given subject is usually set by the judgment of the attending physician. However, in each dose a therapeutically effective amount of oritavancin is typically between about 100 mg and 3000 mg, between about 400 mg to about 1800 mg, between about 500 mg to about 1600 mg, between about 600 mg to about 1400 mg, between about 800 mg to about 1200 mg, between about 1000 mg to about 1400 mg, or between about 1100 mg to about 1400 mg oritavancin.

In certain aspects, a therapeutically effective amount of oritavancin is about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950 or 2000 mg oritavancin. In specific aspects, the dose contains about 800, 900, 1000, 1100 or 1200 mg oritavancin.

In certain other aspects, a therapeutically effective amount of oritavancin is at least about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950 or 2000 mg oritavancin. In a preferred aspect, the dose contains at least about 400 mg oritavancin.

Depending on the means of administration, the dosage may be administered all at once, such as with an oral formulation in a capsule, or slowly over a period of time, such as with an intravenous administration. For slower means of administration, the administering period can be a matter of minutes, such as about 5, 10, 15, 20, 25, 30, 35, 40, 45, 50, 55, 60, 65, 70, 75, 80, 85, 90, 95, 100, 105, 110, 115, 120 or more minutes, or a period of hours, such as about 0.5, 1, 1.5, 2, 2.5, 3, 3.5, 4, 4.5, 5 or more hours. The administration of the dose may be interrupted, such as where the dose is administered via intravenous infusion and the dose is divided into two or more infusion bags. Under such circumstances, the administration of the dose may be interrupted while the infusion bags are changed.

### Oritavancin or a Salt Thereof for Use in Methods of Prophylaxis

There is described herein oritavancin or a salt thereof for use in methods of providing prophylaxis for one or more of bacteremia, osteomyelitis, or endocarditis in a subject by administering oritavancin, or a pharmaceutically acceptable salt thereof, preferably formulated as a pharmaceutical composition to a subject at risk for developing bacteremia, osteomyelitis, or endocarditis. There is described herein oritavancin or a salt thereof for use inamethod of providing prophylaxis foroneormoreof bacteremia, osteomyelitis, or endocarditis in a subject, comprising administering a therapeutically effective amount of a pharmaceutical composition comprising oritavancin, or a pharmaceutically acceptable salt thereof, and a pharmaceutically acceptable carrier or diluent, over a course of prophylaxis to a subject at risk of developing bacteremia, osteomyelitis, or endocarditis, thereby providing prophylaxis for bacteremia, osteomyelitis, or endocarditis in a subject. A second therapeutic agent may be administered to the subject in conjunction with the pharmaceutical composition comprising oritavancin. In an aspect of this disclosure, the subject is at risk for developing bacteremia. In an aspect of this disclosure, the subject is at risk for developing both bacteremia and osteomyelitis. In an aspect of this disclosure, the subject is at risk for developing both bacteremia and endocarditis. In an aspect of this disclosure, the subject is at risk for developing each of bacteremia, osteomyelitis, and endocarditis.

Many physicians believe that humans should be considered for antibiotic prophylaxis before a surgical procedure, a dental procedure or invasive medical procedure to mitigate the potential for an infection resulting from ineffective sterility during the procedure. Oritavancin may therefore be used as a replacement for, or in addition to, prophylactic antibiotics in this situation. For instance, oritavancin and/or pharmaceutical compositions of the disclosure may be administered to a subject to achieve a systemic and/or local effect against relevant bacteria shortly before an invasive medical treatment, such as surgery or insertion of an in-dwelling device (e.g. joint replacement (hip, knee, shoulder, etc.)). Treatment may be repeated after the invasive medical treatment, such as post-operatively or during the in-body time of the device.

The term "prophylaxis" means a reduction in the likelihood that a bacterial infection or a disease condition associated with a bacterial infection will develop in a subject, preferably a human. In particular, the term is related to the administration of oritavancin to a subject to reduce the likelihood of the occurrence of a bacterial infection, such as bacterial infection that may occur during or following a surgery, a dental procedure or invasive medical procedure. For example, one can reduce the likelihood of a bacterial infection in a subject by administering oritavancin before exposure to bacteria. The prophylaxis may be about a reduction of at least about 100%, 99%, 98%, 97%, 96%, 95%, 90%, 80%, 70%, 60%, 50%, 40%, 30%, 20%, 10%, 5% or 1% in the subject, versus a subject to which oritavancin has not been administered. The prophylaxis may last in the subject for at least about 0.5, 1, 2, 3, 4, 5, 6, 7, 8, 9, 10, 11, 12, 13, 14, 15, 16, 17, 18, 19, 20, 22, 23 or 24 hours, or 2, 3, 4, 5, 6, 7, 8, 9, 10, 12, 14, 16, 18, 20, 22, 24, 26, 28, 30 or more days after administration of oritavancin. In an alternative aspect, prophylaxis is maintained for the duration of a surgical procedure, a dental procedure or an invasive medical procedure.

In disclosureprovidedherein, prophylaxis (i.e., a reduction in the likelihood that a bacterial infection or a disease condition associated with a bacterial infection will develop in a subject) is maintained for at least about 4, 8 or 12 hours. In adisdosureprovidedherein,the reduction in the likelihood that a bacterial infection or a disease condition associated with a bacterial infection will develop in a subject is a reduction of at least about 90% for at least about 4 hours. In a disclosure provided herein, the reduction in the likelihood that a bacterial infection or a disease condition associated with a bacterial infection will develop in a subject is a reduction of at least about 95% for at least about 4 hours.

Oritavancin or a pharmaceutical composition comprising the compound may be administered within 10, 9, 8, 7, 6, 5, 4, 3, 2, or 1 day, or within 24, 22, 20, 18, 16, 14, 12, 10, 9, 8, 7, 6, 5, 4, 3, 2, 1 or 0.5 hour prior to when a subject will potentially be exposed to bacteria, such a prior to surgery or a dental procedure, thereby permitting an advisable systemic or local presence of oritavancin, preferably in the areas potentially exposed to bacterial contamination during the surgical procedure.

The therapeutically effective amount of oritavancin in each dosage for prophylaxis will vary depending, for example, upon the physical characteristics of the subject, the procedure to which the subject will be subjected, the identity of the bacteria that could potentially cause infection, the formulation and the means used to administer the drug. The specific dose for a given subject is usually set by the judgment of the attending physician. However, in each dose a therapeutically effective amount of oritavancin is typically between about 100 mg and 3000 mg, between about 400 mg to about 1800 mg, between about 500 mg to about 1600 mg, between about 600 mg to about 1400 mg, between about 800 mg to about 1200 mg, between about 1000 mg to about 1400 mg, or between about 1100 mg to about 1400 mg oritavancin.

In certain aspects, a therapeutically effective amount of oritavancin is about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950 or 2000 mg oritavancin. In specific aspects, the dose contains about 800, 900, 1000, 1100 or 1200 mg oritavancin.

In certain other aspects, a therapeutically effective amount of oritavancin is at least about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950 or 2000 mg oritavancin. In a preferred aspect, the dose contains at least about 400 mg oritavancin.

Depending on the means of administration, the dosage may be administered all at once, such as with an oral formulation in a capsule, or slowly over a period of time, such as with an intravenous administration. For slower means of administration, the administering period can be a matter of minutes, such as about 5, 10, 15, 20, 25, 30, 35, 40, 45, 50, 55, 60, 65, 70, 75, 80, 85, 90, 95, 100, 105, 110, 115, 120 or more minutes, or a period of hours, such as about 0.5, 1, 1.5, 2, 2.5, 3, 3.5, 4, 4.5, 5 or more hours. The administration of the dose may be interrupted, such as where the dose is administered via intravenous infusion and the dose is divided into two or more infusion bags. Under such circumstances, the administration of the dose may be interrupted while the infusion bags are changed.

### Oritavancin or a Salt Thereof for Use in Methods of Preventing

There is described herein oritavancin or a salt thereof for use in methods of preventing one or more of bacteremia, osteomyelitis, or endocarditis in a subject by administering oritavancin, or a pharmaceutically acceptable salt thereof, preferably formulated as a pharmaceutical composition to a subject at risk of developing bacteremia, osteomyelitis, or endocarditis. Thus, the disclosure includes a therapeutically-effective amount of oritavancin or a salt thereof for use in a method of preventing one or more of bacteremia, osteomyelitis, or endocarditis in a subject, comprising administering a therapeutically effective amount of a pharmaceutical composition comprising oritavancin, or a pharmaceutically acceptable salt thereof, and a pharmaceutically acceptable carrier or diluent to a subject at risk of developing bacteremia, osteomyelitis, or endocarditis. A second therapeutic agent may be administered to the subject in conjunction with the pharmaceutical composition comprising oritavancin. In an aspect of this disclosure, the subject is at risk for developing bacteremia. In an aspect of this disclosure, the subject is at risk for developing both bacteremia and osteomyelitis. In an aspect of this disclosure, the subject is at risk for developing both bacteremia and endocarditis. In an aspect of this disclosure, the subject is at risk for developing each of bacteremia, osteomyelitis, and endocarditis.

The terms "prevent" and "prevention" mean blocking or stopping a bacterial infection or a disease condition associated with a bacterial infection from developing in a subject, preferably a human. Such methods may be practiced, for example, on subjects having a higher risk for bacterial infection than the general population, including patients undergoing treatment for bacterial infections whereby normal gut flora is inhibited by antimicrobial therapy, patients with impaired immune function (e.g., immunoglobulin deficiency, splenic dysfunction, splenectomy, HIV infection, impaired leukocyte function, hemoglobinopathies), the elderly (Loo et al., 2005. NEJM 353:2442), people with certain malignancies (e. g., multiple myeloma, chronic lympocytic leukemia, lymphoma), people at increased occupational risk (e.g., public services workers, such a fire, water, sanitary, police, medical, and laboratory workers, hospital workers), people in closed populations (e.g., prisons, military, nursing homes) and others that have immunological deficiencies that might enhance their susceptibility to bacterial infection. The prevention may last in the subject for at least about 0.5, 1, 2, 3, 4, 5, 6, 7, 8, 9, 10, 11, 12, 13, 14, 15, 16, 17, 18, 19, 20, 25, 30, 35, 40, 45, 50 or more days after administration of oritavancin.

In one aspect of the disclosure, the prevention lasts at least about 24 hours in the subject. In another aspect of the disclosure, the prevention lasts at least about 72 hours in the subject. In further aspect of the disclosure, the prevention lasts at least about 144 hours in the subject.

Oritavancin or a pharmaceutical composition comprising the compound may be administered within 10, 9, 8, 7, 6, 5, 4, 3, 2, or 1 day, or within 24, 22, 20, 18, 16, 14, 12, 10, 9, 8, 7, 6, 5, 4, 3, 2, 1 or 0.5 hour prior to when a subject will potentially be exposed to bacteria, such prior to contact by military personnel with a material suspected of containing a particular bacteria.

The therapeutically effective amount of oritavancin in each dosage for prevention will vary depending, for example, upon the physical characteristics of the subject, the identity of the bacteria to which the subject may be exposed, the formulation and the means used to administer the drug. The specific dose for a given subject is usually set by the judgment of the attending physician. However, in each dose a therapeutically effective amount of oritavancin is typically between about 100 mg and 3000 mg, between about 400 mg to about 1800 mg, between about 500 mg to about 1600 mg, between about 600 mg to about 1400 mg, between about 800 mg to about 1200 mg, between about 1000 mg to about 1400 mg, or between about 1100 mg to about 1400 mg oritavancin.

In certain aspects, a therapeutically effective amount of oritavancin is about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950 or 2000 mg oritavancin. In specific aspects, the dose contains about 800, 900, 1000, 1100 or 1200 mg oritavancin.

In certain other aspects, a therapeutically effective amount of oritavancin is at least about 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950 or 2000 mg oritavancin. In a preferred aspect, the dose contains at least about 400 mg oritavancin.

Depending on the means of administration, the dosage may be administered all at once, such as with an oral formulation in a capsule, or slowly over a period of time, such as with an intravenous administration. For slower means of administration, the administering period can be a matter of minutes, such as about 5, 10, 15, 20, 25, 30, 35, 40, 45, 50, 55, 60, 65, 70, 75, 80, 85, 90, 95, 100, 105, 110, 115, 120 or more minutes, or a period of hours, such as about 0.5, 1, 1.5, 2, 2.5, 3, 3.5, 4, 4.5, 5 or more hours. The administration of the dose may be interrupted, such as where the dose is administered via intravenous infusion and the dose is divided into two or more infusion bags. Under such circumstances, the administration of the dose may be interrupted while the infusion bags are changed.

As used herein, the terms "dose", "unit dose", "dosage", "effective dose" and related terms refer to physically discrete units that contain a predetermined quantity of active ingredient calculated to produce a desired therapeutic effect. A single dose is thus a predetermined quantity of oritavancin that is administered to a subject. Preferably,
the oritavancin is formulated as a pharmaceutical composition for administration to the subject.

As used herein, the term "course of therapy" depends on the particular method of the disclosure, however the term generally means the period of time within which or over which a selected goal is achieved. In terms of a therapeutically-effective amount of oritavancin or a salt thereof for use in methods of treatment, the course of therapy is the time period which is required to achieve treatment of the bacterial infection in the subject. In terms of a therapeutically-effective amount of oritavancin or a salt thereof for use in methods of prophylaxis, the course of therapy is the period of time over which prophylaxis for a bacterial infection is achieved. In terms of a therapeutically-effective amount of oritavancin or a salt thereof for use in methods of prevention, the course of therapy is the period of time over which prevention from a bacterial infection is achieved.

There is described herein oritavancin or a salt thereof for use in the treatment, prevention and/or prophylaxis of bacterial infections, and therapeutic and prophylactic methods against other diseases caused by or related to bacterial infection, including but not limited to otitis, conjunctivitis, pneumonia, bacteremia, sinusitis, pleural empyema and endocarditis, intravascular or endothelial infections, osteomyelitis and meningitis. In such disclosure, oritavancin is administered to a subject in an amount sufficient to provide a therapeutic effect and thereby prevent or treat the infection of the subject.

### IV. Examples

### Example 1 - Treatment of Bacteremia In vivo

In response to a compassionate use request, oritavancin was administered to a subject having bacteremia.

The demographics of the subject included the following: gender - male; age - 73; race - Caucasian; weight - 190 lb; height- 70 inches. The subject's medical history included the following: CAD s/p CABG 8/08 (SVG to OM1, SVG to PDA); severe AS s/p bioprosthetic AVR 8/08; lumbar discectomy and fusion Feb. 2011, hardware removed Jan. 11, 2013; PVD S/p bilateral CEA 2010; CKD s/p renal artery stenting Dec. 2011, Baseline Cr approx 3.0 s; HTN; CHF; HLD; mild pulmonary fibrosis; BPH; GERD; arthritis.

The treatment history for the subject included the following.
- 2012 July: presented with months of malaise and fatigue; blood culture positive for VRE (linezolid susceptible, daptomycin MIC = 4 - borderline susceptible, tigecycline susceptible); TEE negative; treatment: daptomycin and tigecycline for 6 weeks, line was pulled because *Pseudomonal* infection developed; PO linezolid for 3 weeks and was discontinued in late Sept. 2012 due to nausea.
- 2012 December: presented with back pain, Positive blood culture for VRE again; treatment: daptomycin and tigecycline, blood culture remain positive 9 daptomycin MIC=6, televancin resistant; blood culture was cleared on tigecycline and linezolid. TEE negative; CT/PET showed evidence of activity at spinal hardware; hardware removed on Jan. 11, 2013; the plan was for at least 8 weeks of antibiotic therapy.
- 2013 Feb. 4: readmitted due to severe intractable nausea, anorexia, thrombocytopeania and anemia.
- Treatment as of Feb. 7, 2013: heparin 5,000 units SC Q8H; tigecycline 50 mg IV Q12H; linezolid 600 mg IV Q12H; metoprolol tartrate 6.25 mg PO Q6H; ondansetron HCL (chemo N/V) 4 mg IV Q6H; oxycodone 5-10 mg PO Q4H PRN; Tylenol 650 mg PO Q4H PRN headache.

The subject was diagnosed with: 1) vancomycin-resistant enterococcal bacteremia with intolerance of other antibiotics; and 2) possible spinal osteomyelitis. • Treatment as of Feb. 8, 2013: oritavancin (1200 mg IV, Q2D x 3 times, then QW x 5 times).

The results reported on Feb. 11, 2013 included the following.
- Infusion started Feb. 8, 2013; second dose was given Feb. 10, 2013.
- Patient is doing fine. He had hospital-related delirium but this was also a pre-existing condition and he is now getting better.
- His renal function seems to be stable (last post-dose creatinine was 3.1; pre-treatment levels were between 2.8 to 3).
- Patient remains hospitalized. Attending physician will consider sending him home after the third dose (Feb. 12, 2013), most likely toward this weekend and then schedule him to return for the remaining dosing.
- Last blood culture was Feb. 4, 2013 (negative). Attending physician will take blood sample for culture Feb. 11, 2013 and weekly thereafter.
- PK samples have been taken.

The results reported on Feb. 20, 2013 included the following.
- He is tolerating oritavancin well without the adverse events from his previous antibiotics. He feels much better and he is "extremely happy."
- His anemia was improved (because he eats better).
- His T wave inversion (pre-existing) has improved.
- His hospital-related delirium (pre-existing) has resolved
- His creatinine went down largely (from 3.1 to 1.6) and is at the lowest level since he saw attending physician.
- He has improvement in many lab results.
- The Feb. 11, 2013 blood culture was negative; the last blood culture (samples taken at 1:30 pm Feb. 19, 2013) is so far negative (culture duration is 5 days).
- Attending physician kept the infusion time at 4 hours.

The concentrations are consistent with expectations from modeling (modeled oritavancin concentration-time profile is attached), with oritavancin attaining peaks of about 120-130 ug/mL and troughs of around 5-10 ug/mL for the loading phase and of around 5 ug/mL for the weekly dosing phase. Importantly, there is no evidence of substantial accumulation in the weekly dosing phase.

| **Sample Name** | **Oritavancin (µg/mL)** |
|---|---|
| MDCO-ORI-COM 417490000001 00001 A Peak - First Dose PLM-1 | 114.793 |
| MDCO-ORI-COM 417490000002 00001 A Trough - First Dose PLM-1 | 4.272 |
| MDCO-ORI-COM 417490000003 00001 A Trough - Second Dose PLM-1 | 7.614 |
| MDCO-ORI-COM 417490000004 00001 A Peak - Third Dose PLM-1 | 133.581 |
| MDCO-ORI-COM 417490000005 00001 FL13-141 trough, third dose PLM-1 | 3.775 |
| MDCO-ORI-COM 417490000006 00001 FL13-141 trough, fourth dose PLM-1 | 4.256 |
| MDCO-ORI-COM 417490000007 00001 FL13-141 trough, fifth dose PLM-1 | 4.584 |
| MDCO-ORI-COM 417490000008 00001 FL13-141 trough, sixth dose PLM-1 | 4.489 |

### Example 2 - Activity against Sources of Bacteremia In vitro

Gram-positive organisms (staphylococci, enterococci and streptococci) are largely responsible for infections in the community and nosocomial settings. More often than appreciated, these infections may involve a causative agent exhibiting a multidrug-resistant (MDR) phenotype, and when associated with critically ill patients may provide a greater challenge for antimicrobial therapies. These serious infections are also associated with increased morbidity and mortality, and create a significant economic burden. Moreover, the therapeutic options for treating these serious Gram-positive conditions have become limited since few agents have been recently approved for clinical use.

Oritavancin is a semisynthetic bactericidal lipoglycopeptide in Phase 3 clinical development for the treatment of patients with acute bacterial skin and skin structure infections (ABSSSI). This drug has demonstrated broad *in vitro* activity against Gram-positive pathogens, including MDR strains of methicillin-resistant *Staphylococcus aureus* (MRSA), other Staphylococcus spp., streptococci, and enterococci, including strains with elevated vancomycin MIC values. In this study, oritavancin and comparator agent activities were evaluated against S. *aureus,* coagulase-negative staphylococci (CoNS), *Enterococcus faecalis* and *Enterococcus faecium* recovered from blood specimens among hospitalized patients in European Union and other surrounding countries during the international surveillance program for oritavancin (2010-2012).

### Methods

**Bacterial strain collection.** A total of 2,904 clinical strains responsible for documented bacteremia among unique hospitalized patients in 38 hospitals in 11 European Union countries, Israel, Russia, Turkey and Ukraine were included in this study (2010-2012). Isolates were submitted to a central monitoring laboratory (JMI Laboratories, North Liberty, Iowa, USA), following previously established protocols. Bacterial species identification was performed by using an automated system (Vitek^{®}2; bioMérieux, Hazelwood, Missouri, USA) or conventional biochemical algorithms, as required.

**Antimicrobial susceptibility testing methods.** Isolates were tested for susceptibility by broth microdilution methods following the Clinical and Laboratory Standards Institute (CLSI; M07-A9, 2012) document. Susceptibility testing was performed in cation-adjusted Mueller-Hinton broth (CA-MHB) using dry-form panels manufactured by Thermo Fisher Scientific, formerly TREK Diagnostics Systems/Sensititre (Cleveland, Ohio, USA). These panels provide results equivalent to the CLSI-approved broth microdilution method supplemented with 0.002% polysorbate-80.

Quality assurance was performed by concurrent testing of CLSI-recommended (M100-S23, 2013) strains: *E. faecalis* ATCC 29212 and *S. aureus* ATCC 29213. Interpretation of comparator MIC results was in accordance with published CLSI (M100-S23) and European Committee on Antimicrobial Susceptibility Testing (EUCAST, 2013) criteria. *E. faecalis* and *E*. *faecium* displaying vancomycin and teicoplanin MIC values of >4 and >2 mg/L, respectively, were considered as VanA-phenotype.

### Results

Oritavancin exhibited MIC₅₀ and MIC₉₀ results of 0.03 and 0.06 mg/L, respectively when tested against a collection of staphylococcal clinical isolates, regardless of the methicillin susceptibility pattern, inhibiting all strains at ≤0.25 mg/L (Table 1). Oritavancin MIC₅₀, MIC₉₀ and modal MIC results against S. *aureus* isolates with decreased susceptibility to vancomycin (MIC = 2 mg/L) were two-fold higher than those obtained against strains with vancomycin MIC ≤1 mg/L (Table 1). When tested against MRSA strains, oritavancin (MIC_{50/90}, 0.03/0.06 mg/L) was eight-fold more potent than daptomycin (MIC_{50/90}, 0.25/0.5 mg/L) and 16- to 32-fold more active than vancomycin (MIC_{50/90}, 1/1 mg/L) or linezolid (MIC_{50/90}, 1/1 mg/L; Table 2).

A subset of CoNS clinical isolates causing bacteraemia and displaying a decreased susceptibility to teicoplanin (MIC >8 mg/L), demonstrated oritavancin MIC results (highest MIC value, 0.25 mg/L) two-fold higher than strains with teicoplanin MIC <4 mg/L (Table 1). Oritavancin exhibited MIC₅₀ and MIC₉₀ results (0.03 and 0.06 mg/L, respectively) eight- and 16-fold lower than daptomycin (MIC_{50/90}, 0.25/0.5 mg/L) and linezolid (MIC_{50/90}, 0.5/1 mg/L) when tested against CoNS isolates, respectively (Table 2). Moreover, the oritavancin MIC values obtained against CoNS were 32- to 64-fold lower than vancomycin (MIC_{50/90}, 2/2 mg/L).

Oritavancin (MIC_{50/90}, 0.015/0.06 mg/L) was very active against *E. faecalis* strains, as were ampicillin (MIC_{50/90}, ≤1/2 mg/L; 99.6% susceptible), vancomycin (MIC_{50/90}, 1/2 mg/L; 99.1% susceptible), teicoplanin (99.1% susceptible), daptomycin (MIC_{50/90}, 1/1 mg/L; 100% susceptible) and linezolid (MIC_{50/90}, 1/2 mg/L; 100% susceptible). Oritavancin (MIC_{50/90}, 0.015/0.06 mg/L) was at least two-fold less active when tested against vancomycin-resistant (VanA-type) *E. faecium* strains compared with the wild-type vancomycin-susceptible population (MIC_{50/90}, ≤0.008/≤0.008 mg/L; Tables 1 and 2). However, oritavancin inhibited all VanA-phenotype *E. faecium* strains at ≤0.12 mg/L. Daptomycin (MIC_{50/90}, 2/2 mg/L; 100% susceptible) and linezolid (MIC_{50/90}, 1/2 mg/L; 96.2% susceptible) showed good coverage against VanA-phenotype *E. faecium.* However, oritavancin demonstrated MIC₅₀ and MIC₉₀ results 32- to 64-fold lower than these comparators (Table 2).

**Table 1. MIC distribution of oritavancin tested against Gram-positive clinical isolates, including resistant subsets, causing bacteraemia as part of the 2010 - 2012 international oritavancin surveillance programme.**

| Organism (no. tested)/ | | MIC (mg/L) | | Number (cumulative %) inhibited at MIC (mg/L)^{a} | | | | | | |
|---|---|---|---|---|---|---|---|---|---|---|
| | Subset | 50% | 90% | ≤0.008 | 0.015 | 0.03 | 0.06 | 0.12 | 0.25 | 0.5 |
| *S. aureus* (1,498) | | | | | | | | | | |
| | Methicillin-susceptible (1,126) | 0.03 | 0.06 | 28(2.5) | 247(24.4) | **458(65.1)** | 297(91.5) | 78(98.4) | 18(100.0) | |
| | Methicillin-resistant (372) | 0.03 | 0.06 | 5(1.3) | 86(24.5) | **180(72.9)** | 68(91.1) | 27(98.4) | 6(100.0) | |
| | Vancomycin MIC ≤1 mg/L (1,479) | 0.03 | 0.06 | 33(2.2) | 332(24.7) | **635(67.6)** | 354(91.5) | 102(98.4) | 23(100.0) | |
| | Vancomycin MIC = 2 mg/L (19) | 0.06 | 0.12 | 0(0.0) | 1(5.3) | 3(21.1) | **11(78.9)** | 3(94.7) | 1(100.0) | |

| Coagulase-negative staphylococci (646) | | | | | | | | | | |
|---|---|---|---|---|---|---|---|---|---|---|
| | Methicillin-susceptible (137) | 0.03 | 0.06 | 37(27.0) | 28(47.5) | **50(83.9)** | 21(99.3) | 1(100.0) | | |
| | Methicillin-resistant (509) | 0.03 | 0.06 | 100(19.7) | 77(34.8) | **181(70.3)** | 114(92.7) | 33(99.2) | 4(100.0) | |
| | Teicoplanin MIC ≤4 mg/L (574) | 0.03 | 0.06 | 136(23.7) | 104(41.8) | **216(79.4)** | 99(96.7) | 19(100.0) | | |
| | Teicoplanin MIC ≥8 mg/L (72) | 0.06 | 0.12 | 1(1.4) | 1(2.8) | 15(23.6) | **36(73.6)** | 15(94.4) | 4(100.0) | |
| *E. faecalis*^{b} (445) | | 0.015 | 0.06 | 104(23.4) | **204(69.2)** | 89(89.2) | 30(96.0) | 13(98.9) | 4(99.8) | 1(100.0) |
| *E*. faecium^{c} (315) | | ≤0.008 | 0.015 | **269(85.4)** | 25(93.3) | 12(97.1) | 6(99.1) | 3(100.0) | | |
| | Vancomycin-susceptible (257) | ≤0.008 | ≤0.008 | **251(97.7)** | 6(100.0) | | | | | |
| | VanA-phenotype^{d} (53) | 0.015 | 0.06 | 13(24.5) | **19(60.4)** | 12(83.0) | 6(94.3) | 3(100.0) | | |

| | | | | | | | | | | |
|---|---|---|---|---|---|---|---|---|---|---|
| a. Modal MIC values are shown in bold. b. Includes four VanA-phenotype strains displaying oritavancin MIC values of 0.12 - 0.5 mg/L. c. Includes five VanB-phenotype strains displaying vancomycin and teicoplanin MIC values of >4 and ≤2 mg/L, respectively. All oritavancin MIC values for these VanB strains were at ≤0.008 mg/L. d. VanA-phenotype = vancomycin and teicoplanin MIC values of >4 and >2 mg/L, respectively (EUCAST criteria for non-susceptibility). | | | | | | | | | | |

**Table 2. Antimicrobial activity of oritavancin and comparator agents tested against Gram-positive clinical isolates, including resistant subsets, causing bacteraemia as part of the 2010 - 2012 international oritavancin surveillance programme.**

| Organism (number tested) | | Range | MIC (mg/L) | | % Susceptible/Resistant^{a} | |
|---|---|---|---|---|---|---|
| | Antimicrobial agent | | 50% | 90% | CLSI | EUCAST |
| *S. aureus* | | | | | | |
| | Methicillin-resistant (1,708) | | | | | |
| | Oritavancin | ≤0.008 - 0.25 | 0.03 | 0.06 | -^{b}/- | -/- |
| | Vancomycin | 0.25 - 2 | 1 | 1 | 100.0/0.0 | 100.0/0.0 |
| | Teicoplanin | ≤2 | ≤2 | ≤2 | 100.0/0.0 | 100.0/0.0 |
| | Daptomycin | 0.12 - 1 | 0.25 | 0.5 | 100.0/- | 100.0/0.0 |
| | Linezolid | 0.25 - 2 | 1 | 1 | 100.0/0.0 | 100.0/0.0 |
| | Erythromycin | ≤0.25 - >4 | >4 | >4 | 29.6/68.0 | 29.8 / 69.1 |
| | Clindamycin | ≤0.25 - >2 | ≤0.25 | >2 | 59.1 / 40.3 | 58.9/40.9 |
| | Tetracycline | ≤0.25 - >8 | ≤0.25 | >8 | 84.9 / 14.2 | 84.4 / 15.1 |
| | Levofloxacin | ≤0.5 - >4 | >4 | >4 | 8.6 / 90.3 | 8.6 / 90.3 |
| | Trimethoprim/sulfamethoxazole | ≤0.5 - >4 | ≤0.5 | ≤0.5 | 97.8 / 2.2 | 97.8 / 2.2 |

| Coagulase-negative staphylococci (646) | | | | | | |
|---|---|---|---|---|---|---|
| | Oritavancin | ≤0.008 - 0.25 | 0.03 | 0.06 | - / - | - / - |
| | Oxacillin | ≤0.25 - >2 | >2 | >2 | 21.2 / 78.8 | 21.2 / 78.8 |
| | Vancomycin | 0.25 - 4 | 2 | 2 | 100.0/0.0 | 100.0/0.0 |
| | Teicoplanin | ≤2 - >8 | ≤2 | 8 | 97.7/0.8 | 88.9/ 11.1 |
| | Daptomycin | ≤0.06 - 2 | 0.25 | 0.5 | 99.8/- | 99.8/0.2 |
| | Linezolid | ≤0.12 - >8 | 0.5 | 1 | 99.2/0.8 | 99.2/0.8 |
| | Erythromycin | ≤0.25 - >4 | >4 | >4 | 34.9 / 65.1 | 34.9 / 65.1 |
| | Clindamycin | ≤0.25 - >2 | ≤0.25 | >2 | 73.0/26.0 | 70.9/27.0 |
| | Tetracycline | ≤0.25 - >8 | 1 | >8 | 83.1 / 15.2 | 68.7 / 19.3 |
| | Levofloxacin | ≤0.5 - >4 | 4 | >4 | 39.2 / 56.2 | 39.2 / 56.2 |
| | Trimethoprim/sulfamethoxazole | ≤0.5 - >4 | 1 | >4 | 56.5 / 43.5 | 56.5/24.1 |

| *E. faecalis^{c}* (445) | | | | | | |
|---|---|---|---|---|---|---|
| | Oritavancin | ≤0.008 - 0.5 | 0.015 | 0.06 | - / - | - / - |
| | Ampicillin | ≤1 - 8 | ≤1 | 2 | 100.0/0.0 | 99.6/0.0 |
| | Vancomycin | 0.5 - >16 | 1 | 2 | 99.1 / 0.9 | 99.1 / 0.9 |
| | Teicoplanin | ≤2 - >8 | ≤2 | ≤2 | 99.1 / 0.9 | 99.1 / 0.9 |
| | Daptomycin | ≤0.06 - 4 | 1 | 1 | 100.0 / - | -/- |
| | Linezolid | 0.5-2 | 1 | 2 | 100.0/0.0 | 100.0/0.0 |
| | Erythromycin | ≤0.25 - >4 | >4 | >4 | 8.1 / 54.8 | -/- |
| | Tetracycline | ≤0.25 - >8 | >8 | >8 | 26.5/73.5 | -/- |
| | Levofloxacin | ≤0.5 - >4 | 1 | >4 | 64.3/35.3 | -/- |

| *E. faecium* | | | | | | |
|---|---|---|---|---|---|---|
| Vancomycin-susceptible (257) | | | | | | |
| | Oritavancin | ≤0.008 - 0.015 | ≤0.008 | ≤0.008 | - / - | - / - |
| | Ampicillin | ≤1 - >8 | >8 | >8 | 5.8 / 94.2 | 5.4 / 94.2 |
| | Vancomycin | 0.5-4 | 1 | 1 | 100.0/0.0 | 100.0/0.0 |
| | Teicoplanin | <2 - 4 | ≤2 | ≤2 | 100.0/0.0 | 99.6 / 0.4 |
| | Daptomycin | 0.12-4 | 2 | 2 | 100.0 / - | -/- |
| | Linezolid | 0.25 - 2 | 1 | 1 | 100.0/0.0 | 100.0/0.0 |
| | Erythromycin | ≤0.25 - >4 | >4 | >4 | 3.5/91.4 | -/- |
| | Tetracycline | ≤0.25 - >8 | 0.5 | >8 | 56.0/43.2 | -/- |
| | Levofloxacin | 1 - >4 | >4 | >4 | 5.8 / 89.1 | -/- |

| Vancomycin-resistant (VanA-phenotype) (53) | | | | | | |
|---|---|---|---|---|---|---|
| | Oritavancin | ≤0.008 - 0.12 | 0.015 | 0.06 | - / - | - / - |
| | Ampicillin | >8 | >8 | >8 | 0.0/ 100.0 | 0.0 / 100.0 |
| | Vancomycin | >16 | >16 | >16 | 0.0/100.0 | 0.0/100.0 |
| | Teicoplanin | >8 | >8 | >8 | 0.0 / 96.2 | 0.0 / 100.0 |
| | Daptomycin | 0.25 - 4 | 2 | 2 | 100.0 / - | -/- |
| | Linezolid | 0.5-8 | 1 | 2 | 96.2/3.8 | 96.2/3.8 |
| | Erythromycin | 4 - >4 | >4 | >4 | 0.0 / 98.1 | -/- |
| | Tetracycline | ≤0.25 - >8 | 0.5 | >8 | 58.5 / 41.5 | -/- |
| | Levofloxacin | >4 | >4 | >4 | 0.0/ 100.0 | -/- |

| | | | | | | |
|---|---|---|---|---|---|---|
| ^{a} Breakpoint criteria according to CLSI (M100-S23, 2013) and EUCAST (2013). ^{b} Breakpoints not available. ^{c} Includes four VanA-phenotype strains. | | | | | | |

### Discussion

Oritavancin demonstrated high *in vitro* potency when tested against this contemporary (2010-2012) collection of Gram-positive clinical isolates causing bacteraemia among hospitalized patients in the European Union and surrounding countries. The *in vitro* activity of oritavancin tested against staphylococci was not affected by the methicillin-resistance phenotype. In addition, oritavancin MIC results were not significantly (>two-fold) affected by the glycopeptide (vancomycin and teicoplanin) susceptibilities and inhibited all staphylococci, *E*. *faecalis* and *E. faecium,* including resistant subsets, at ≤0.25, ≤0.5 and ≤0.12 mg/L, respectively.

### Citations

Arhin FF, Moeck G, Draghi DC, Pillar CM, Sahm DF (2010). Longitudinal analysis of the in vitro activity profile of oritavancin and comparator glycopeptides against Gram-positive organisms from Europe: 2005-2008. Int J Antimicrob Agents 36: 474-476.

Clinical and Laboratory Standards Institute (2012). M07-A9. Methods for dilution antimicrobial susceptibility tests for bacteria that grow aerobically; approved standard: ninth edition. Wayne, PA: CLSI.

Clinical and Laboratory Standards Institute (2013). M100-S23. Performance standards for antimicrobial susceptibility testing: 23rd informational supplement. Wayne, PA: CLSI.

European Committee on Antimicrobial Susceptibility Testing (2013). Breakpoint tables for interpretation of MICs and zone diameters. Version 3.0, January 2013. Available at: eucast.org/clinical_breakpoints/. Accessed: January, 2013.

Morrissey I, Seifert H, Canton R, Nordmann P, Stefani S, Macgowan A, Janes R, Knight D, Oritavancin Study Group (2013). Activity of oritavancin against methicillin-resistant staphylococci, vancomycin-resistant enterococci and β-haemolytic streptococci collected from western European countries in 2011. J Antimicrob Chemother 68: 164-167.

### Example 3 - In vitro Activity of Oritavancin (ORI) against Bacteremia Isolates of Enterococci and Beta-Haemolytic Streptococci (BHS)

**Objectives:** Oritavancin (ORI) is a semisynthetic lipoglycopeptide in Phase 3 clinical development for the treatment of acute bacterial skin and skin structure infections. Because bacteremia can be associated with such skin infections, we assessed ORI activity against blood isolates of enterococci and BHS that can be associated with complicated skin infections.

**Methods:** 527 *E. faecalis,* 176 *E. faecium,* and 790 BHS (335 *S. pyogenes* and 455 S. *agalactiae*) clinical, single patient, non-consecutive isolates were collected in 2011-2012 from 46 sites geographically distributed across the United States. The MICs were determined by broth microdilution testing performed in accordance with CLSI M7 and M100 guidelines. From the resulting database, this analysis focused on the activities of ORI, vancomycin (VAN), daptomycin (DAP), and linezolid (LZD) according to specimen source.

**Results:** The table below shows the activity of ORI and comparators against blood and non-blood isolates of enterococci and BHS.

| **Organism** | **Source** | **N** | **MIC ₉₀** | | | |
|---|---|---|---|---|---|---|
| | | | **ORI** | **VAN** | **DAP** | **LZD** |
| *E. faecalis* | Blood | 116 | 0.06 | 2 | 2 | 2 |
| | Non-Blood | 411 | 0.06 | 2 | 2 | 2 |
| *E. faecium* | Blood | 84 | 0.12 | >32 | 4 | 2 |
| | Non-Blood | 92 | 0.12 | >32 | 4 | 2 |
| *S. agalactiae* | Blood | 100 | 0.25 | 0.5 | 0.25 | 1 |
| | Non-Blood | 355 | 0.25 | 0.5 | 0.25 | 1 |
| *S. pyogenes* | Blood | 53 | 0.25 | 0.25 | 0.06 | 1 |
| | Non-Blood | 282 | 0.25 | 0.25 | 0.06 | 1 |

**Conclusions:** Based on MIC₉₀'s ORI exhibited potent activity against all organism groups studied and the level of potency against blood isolates was equivalent to that observed against non-blood isolates. These *in vitro* findings indicate that ORI will provide potent activity against skin pathogens that can also cause associated bacteremia.

### Example 4 - In vitro Activity of Oritavancin (ORI) against Multi-Drug Resistant (MDR) S. aureus (SA) Isolates Associated with Bacteremia, Skin/Wound, and Respiratory and Infections

**Objectives:** Oritavancin (ORI) is a semisynthetic lipoglycopeptide in Phase 3 clinical development for the treatment of acute bacterial skin and skin structure infections. To gain a broader perspective of ORI's in vitro spectrum of activity, we analyzed minimal inhibitory concentrations (MIC) against MDR SA from three primary specimen sources (blood, skin/wound, respiratory). We evaluated ORI activity, relative to that of vancomycin (VAN), daptomycin (DAP), and linezolid (LZD) against currently-circulating Gram-positive pathogens.

**Methods:** 456 clinical, single-patient, non-consecutive, MDR SA isolates from the known specimen sources collected in 2011-2012 were analyzed. MDR was defined as resistant to ≥ 3 of the following: erythromycin, clindamycin, gentamicin, levofloxacin, and trimethoprim/sulfamethoxazole. 382 were MRSA and 74 were MSSA. MICs were determined by microdilution testing in accordance with CLSI M7 and M100 guidelines. Although other comparator drugs were tested, this analysis focuses on the activities of ORI, VAN, DAP, and LZD.

**Results:**

**Table 1. Activity of ORI Against MDR SA According to Specimen Source**

| **Organism** | **Infection Site** | **Drug** | **Range** | **Mode** | **MIC ₅₀** | **MIC ₉₀** |
|---|---|---|---|---|---|---|
| MDR SA | Skin/Wound N=191 | ORI | ≤0.002-0.25 | 0.03 | 0.03 | 0.06 |
| | | VAN | ≤0.25-1 | 0.5 | 0.5 | 1 |
| | | DAP | ≤0.06-1 | 0.5 | 0.5 | 0.5 |
| | | LZD | ≤0.25->4 | 2 | 2 | 2 |
| | Blood n=108 | ORI | 0.015-0.25 | 0.03 | 0.03 | 0.12 |
| | | VAN | 0.5-2 | 0.5 | 0.5 | 1 |
| | | DAP | 0.25-4 | 0.5 | 0.5 | 0.5 |
| | | LZD | 1->4 | 2 | 2 | 2 |
| | Respiratory N=157 | ORI | 0.008-0.25 | 0.03 | 0.03 | 0.06 |
| | | VAN | ≤0.25-1 | 0.5 | 0.5 | 1 |
| | | DAP | 0.12-1 | 0.5 | 0.5 | 0.5 |
| | | LZD | 1->4 | 2 | 2 | 2 |

| | | | | | | |
|---|---|---|---|---|---|---|
| **Conclusions:** Regardless of specimen source, ORI exhibited activity against MDR SA that was several-fold more potent than the other agents analyzed, and ORI's high level of activity was consistent for all MDR SA across the three specimen sources. These findings suggest ORI has strong potential for treating infections caused by problematic MDR SA. | | | | | | |

## Claims

1. A therapeutically-effective amount of oritavancin or a salt thereof for use in a method of treating bacteremia and osteomyelitis in a subject, comprising administering the therapeutically-effective amount of oritavancin or the salt thereof to a subject having bacteremia and osteomyelitis, thereby treating bacteremia and osteomyelitis in a subject.

2. A therapeutically-effective amount of oritavancin or a salt thereof for use according to claim 1, wherein treatment is achieved by administering a single 1200mg dose of oritavancin or a salt thereof to the subject.

3. A therapeutically-effective amount of oritavancin or a salt thereof for use according to claim 1 or claim 2, wherein (i) the bacteremia was resistant to previous antibiotic treatment, (ii) the subject was intolerant to other antibiotics, and/or (iii) the subject also has endocarditis, or both.

4. A therapeutically-effective amount of oritavancin or a salt thereof for use according to claim 1 or claim 2, wherein the bacteremia is caused by a Gram-positive organism, preferably wherein the Gram-positive organism is a multi-drug resistant (MDR) strain of methicillin-resistant *Staphylococcus aureus* (MRSA).

## Patentansprüche

1. Therapeutisch wirksame Menge von Oritavancin oder eines Salzes davon zur Verwendung in einem Verfahren zur Behandlung von Bakteriämie und Osteomyelitis bei einem Probanden, umfassend ein Verabreichen der therapeutisch wirksamen Menge von Oritavancin oder des Salzes davon an einen Probanden mit Bakteriämie und Osteomyelitis, wodurch Bakteriämie und Osteomyelitis bei einem Probanden behandelt wird.

2. Therapeutisch wirksame Menge von Oritavancin oder eines Salzes davon zur Verwendung nach Anspruch 1, wobei eine Behandlung durch Verabreichen einer einzigen 1200-mg-Dosis von Oritavancin oder eines Salzes davon an den Probanden erzielt wird.

3. Therapeutisch wirksame Menge von Oritavancin oder eines Salzes davon zur Verwendung nach Anspruch 1 oder 2, wobei (i) die Bakteriämie gegen eine vorherige Antibiotikabehandlung resistent war, (ii) der Proband gegen andere Antibiotika intolerant war und/oder (iii) der Proband zudem eine Endokardititis hat oder beides.

4. Therapeutisch wirksame Menge von Oritavancin oder eines Salzes davon zur Verwendung nach Anspruch 1 oder 2, wobei die Bakteriämie von einem Gram-positiven Organismus verursacht wird, vorzugsweise wobei der Gram-positive Organismus ein multiresistenter (MDR) Stamm von methicillinresistentem *Staphylococcus aureus* (MRSA) ist.

## Revendications

1. Quantité thérapeutiquement efficace d'oritavancine ou d'un sel de celle-ci destinée à être utilisée dans un procédé de traitement de la bactériémie et de l'ostéomyélite chez un sujet, comprenant l'administration de la quantité thérapeutiquement efficace d'oritavancine ou du sel de celle-ci à un sujet atteint de bactériémie et d'ostéomyélite, traitant ainsi la bactériémie et l'ostéomyélite chez un sujet.

2. Quantité thérapeutiquement efficace d'oritavancine ou d'un sel de celle-ci destinée à être utilisée selon la revendication 1, dans laquelle le traitement est obtenu par l'administration d'une dose unique de 1 200 mg d'oritavancine ou d'un sel de celle-ci au sujet.

3. Quantité thérapeutiquement efficace d'oritavancine ou d'un sel de celle-ci destinée à être utilisée selon la revendication 1 ou la revendication 2, dans laquelle (i) la bactériémie était résistante à un traitement antibiotique antérieur, (ii) le sujet était intolérant à d'autres antibiotiques, et/ou (iii) le sujet était aussi atteint d'endocardite, ou les deux.

4. Quantité thérapeutiquement efficace d'oritavancine ou d'un sel de celle-ci destinée à être utilisée selon la revendication 1 ou la revendication 2, dans laquelle la bactériémie est causée par un organisme à Gram positif, de préférence dans laquelle l'organisme à Gram positif est une souche multi-résistante aux médicaments (MDR) de *Staphylococcus aureus* résistant à la méticilline (MRSA).
